# EUROPÄISCHE PATENTANMELDUNG

(11) **EP 1 671 704 A1**
(43) Veröffentlichungstag der Anmeldung: **21.06.2006**
(21) Anmeldenummer: 05026244.3
(22) Anmeldetag: 01.12.2005
(51) Int. Cl.: B04B 1/20, C13K 1/06, C12P 19/02

(54) **Verfahren und Anlage zur Herstellung von Glukose aus einer Stärkelösung**

(30) Priorität: 17.12.2004 DE 102004060929
(71) Anmelder: Flottweg GmbH & Co. KGaA, 84137 Vilsbiburg (DE)
(72) Erfinder: Bruckmayer, Peter, 84149 Velden-Eberspoint (DE)
(74) Vertreter: Rothkopf, Ferdinand

(57) **Zusammenfassung**

Ein Verfahren zur Herstellung von Glukose aus einer Stärkelösung umfasst erfindungsgemäß die Schritte: Verzuckerung der Stärkelösung (30) durch Zugabe von Enzymen und/oder Säure zu Rohsirup, Separieren von einer im Wesentlichen Proteine und/oder Fette umfassenden Phase (50) aus dem Rohsirup in einer Zentrifugalabtrennvorrichtung (110) und Abscheiden der im Wesentlichen Proteine und/oder Fette umfassenden Phase (50) aus der Zentrifugalabtrennvorrichtung (110) separat vom restlichen Rohsirup.

## Beschreibung

Die Erfindung betrifft ein Verfahren sowie eine Anlage zur Herstellung bzw. Gewinnung von Glukose aus einer Stärkelösung.

Die Umsetzung von Stärke aus einer Stärkelösung in Glukose bzw. verschiedene Zucker ist ein wichtiger Teilbereich der Stärke verarbeitenden Industrie und zugleich eines der wirtschaftlich bedeutendsten Anwendungsfelder der Lebensmitteltechnologie. Die wichtigsten pflanzlichen Stärkelieferanten sind dafür Mais, Kartoffeln und Weizen.

Der Prozess der Glukose- bzw. Dextrosegewinnung läuft in verschiedenen Stufen ab. In einem ersten Schritt wird in der Regel aus einer zuvor hergestellten wässrigen Stärkelösung und weiterem Wasser eine definierte Stärkesuspension hergestellt. Die Stärkesuspension wird durch Zugabe von Dampf, Enzymen und/oder Säure verflüssigt. Daraus entsteht mittels Säure oder enzymatischer Hydrolyse ein Gemisch aus Maltosen (Malzzucker) und Dextrinen (einer Zwischenform von Stärke und Dextrose). Durch eine Zugabe von weiteren Enzymen und/oder Säure wird die Stärkelösung verzuckert. Bei der Stärkeverzuckerung werden die entstandenen Abbauprodukte weiter zu Einfachzucker (Monosaccharide) abgebaut. Es bildet sich ein verzuckerter Rohsirup, welcher ein Gemisch aus Glukose (Traubenzucker) und Fruktose (Fruchtzucker) darstellt. Der Rohsirup enthält aber auch noch wesentliche Anteile an Proteinen, an Fetten und an im Wesentlichen unverzuckerten Bestandteilen, welche zum Herstellen eines fertigen Dextroseproduktes aus dem verzuckerten Rohsirup herausfiltriert werden müssen.

Die Filtration wird in mehrstufigen Ultrafiltrationsanlagen oder Vakuumdrehfiltern mit Kieselgut als Filtermedium durchgeführt und auf diese Weise werden die unerwünschten Nebenprodukte wie Fett und Eiweiß abgeschieden.

Anschließend folgen noch Prozessschritte an einem Ionentauscher, eine Endreinigung und eine Aufkonzentration, bis schließlich durch Mischen verschiedener Dextrosequalitäten die gewünschten Glukose- bzw. Dextroseprodukte im Endzustand hergestellt werden können.

Im oben erläuterten Prozessablauf treten besonders bei der genannten Filtration des verzuckerten Rohsirups Probleme auf, die sich in einem schnellen Leistungsabfall der verwendeten Filter, einer kurzen Standzeit dieser Filter, einer labilen Prozessführung und einem hohen Reinigungsaufwand bzw. einem hohen Verbrauch an Filterhilfsmitteln widerspiegeln.

Bei dem Prozess der Glukose- bzw. Dextrosegewinnung ist ferner zu beachten, dass Zuckerverluste möglichst zu vermeiden sind und daher die unerwünschten Nebenprodukte wie Fett und Eiweiß möglichst ohne einen gleichzeitigen Austrag von Zucker entfernt werden müssen. An die verwendeten mehrstufigen Ultrafiltrationsanlagen und Vakuumdrehfilter werden daher sehr hohe Anforderungen gestellt.

### Zugrundeliegende Aufgabe

Der Erfindung liegt die Aufgabe zugrunde ein Verfahren und eine Anlage zur Herstellung von Glukose aus einer Stärkelösung bereitzustellen, welche im Gegensatz zu bekannten Vorgehensweisen wirtschaftlicher betrieben werden können und, soweit möglich, zugleich zu einer höheren Ausbeute von Glukose bzw. Dextrose im Prozessablauf führen.

### Erfindungsgemäße Lösung

Diese Aufgabe ist erfindungsgemäß mit einem Verfahren zur Herstellung von Glukose aus einer Stärkelösung gelöst, welches die Schritte umfasst: Verzuckerung der Stärkelösung durch Zugabe von Enzymen und/oder Säure zu verzuckerter Stärkelösung bzw. Rohsirup, Separieren von einer im Wesentlichen Proteine und Fette umfassenden Phase aus dem Rohsirup in einer Zentrifugalabtrennvorrichtung und Abscheiden der im Wesentlichen Proteine und/oder Fette umfassenden Phase aus der Zentrifugalabtrennvorrichtung separat vom restlichen Rohsirup.

Mit dem Verfahren gemäß der Erfindung werden die unerwünschten Nebenprodukte wie Fett und Eiweiß aus der verzuckerten Stärkelösung nicht durch einen Filtrationsvorgang abgeschieden, sondern durch eine Zentrifugaltrennung. Es wird erfindungsgemäß also nicht auf das Volumen bzw. die Größe der zu trennenden Partikel des Rohsirups abgestellt, sondern die Partikel werden in Abhängigkeit von ihrem spezifischen Gewicht getrennt.

Eine derartige erfindungsgemäße Trennung führt dazu, dass der Rohsirup im Wesentlichen in drei Phasen aufgeteilt wird, nämlich eine im Wesentlichen Proteine und/oder Fette umfassende Phase, eine im Wesentlichen unverzuckerte Bestandteile umfassende Phase und eine dritte Phase des restlichen Glukosesirups, welche im Wesentlichen Glukose umfasst. Von diesen Phasen kann, wie sich erfindungsgemäß gezeigt hat, durch eine Zentrifugaltrennung insbesondere die im Wesentlichen Proteine und/oder Fette umfassende Phase gut vom restlichen Glukosesirup getrennt werden.

Ferner ist erfindungsgemäß erkannt worden, dass gerade die im Wesentlichen Proteine und/oder Fette umfassende Phase erheblich dafür verantwortlich ist, dass es bei den nachgeschalteten Filtrationsanlagen immer wieder zu Problemen hinsichtlich eines Leistungsabfalls, der Standzeit, der Prozessführung und des Reinigungsaufwands kommt. Durch das erfindungsgemäße Separieren und Abscheiden der im Wesentlichen Proteine und/oder Fette umfassenden Phase aus einer verzuckerten Stärkelösung können hingegen derartige Probleme vermieden werden.

Darüber hinaus können mit dem erfindungsgemäßen Verfahren Zuckerverluste im Prozessablauf der Glukose- bzw. Dextrosegewinnung erheblich verringert werden. Bei Versuchen konnten die Zuckerverluste im Vergleich zu bekannten Verfahren um 50% bis 80% vermindert werden. Diese erhebliche Verminderung von Verlusten basiert auf dem Umstand, dass erfindungsgemäß die unerwünschten Nebenprodukte gezielt in einer einzigen Phase aufkonzentriert werden, welche dann vergleichsweise leicht abgeführt werden kann. Der restliche Glukosesirup weist nachfolgend einen erheblich höheren Volumenkonzentrationsfaktor auf, so dass entsprechend aus diesem Glukosesirup auch erheblich mehr Glukose bzw. Dextrose gewonnen werden kann.

Bei einem Verfahren zur Herstellung von Glukose aus Stärkelösung sind die oben erwähnten unverzuckerten Bestandteile des Rohsirups hinsichtlich der weiteren Prozessschritte im Grunde unschädlich. Bei einer vorteilhaften Weiterbildung des erfindungsgemäßen Verfahrens werden sie aber dennoch separiert und von dem dann im Wesentlichen Glukose umfassenden restlichen Glukosesirup abgeschieden. Auf diese Weise kann der Gesamtleistungsbedarf von nachfolgenden Prozessschritten verringert und die unverzuckerten Bestandteile können dem Prozess gegebenenfalls gezielt in vorgelagerten Prozessschritten wiederzugeführt werden.

Die genannten erfindungsgemäßen Zentrifugalabtrennvorgänge werden besonders vorteilhaft in einer einzigen Zentrifugalabtrennvorrichtung, insbesondere in Gestalt eines einzigen Dekanters durchgeführt. Eine solche Vorgehensweise ist sowohl hinsichtlich der erforderlichen Herstellungs- als auch Betriebskosten besonders günstig. Darüber hinaus führt ein derartiges Verfahren zu einem besonders geringen Bedienungs- und Wartungsaufwand.

Der mit dem erfindungsgemäßen Verfahren behandelte verzuckerte Rohsirup ist derart aufbereitet, dass er besonders vorteilhaft in einer der Zentrifugalabtrennvorrichtung nachfolgenden Filtration gereinigt werden kann. Während der Filtration treten, wie oben erwähnt, im Vergleich zu bekannten Verfahren erfindungsgemäß erheblich geringere Zuckerverluste auf, weil der zu filtrierende restliche Glukosesirup einen erheblich höheren Volumenkonzentrationsfaktor (so genannter VCF) aufweist.

Neben dem erfindungsgemäßen Verfahren ist die der Erfindung zugrunde liegende Aufgabe ferner mit einer Anlage zur Herstellung von Glukose aus einem Stärkelösung gelöst, die mit einer Zugabevorrichtung zum Zugeben von Enzymen und/oder Säure zur Stärkelösung versehen ist, eine Zentrifugalabtrennvorrichtung zum Separieren von einer im Wesentlichen Proteine und/oder Fette umfassenden Phase aus dem verzuckerten Rohsirup aufweist und ferner eine Austragsvorrichtung zum Abscheiden der im Wesentlichen Proteine und/oder Fette umfassenden Phase aus der Zentrifugalabtrennvorrichtung separat von restlichem Glukosesirup umfasst.

Eine derartige erfindungsgemäße Anlage ist in Entsprechung zu dem oben genannten Verfahren vorteilhaft derart weitergebildet, dass sie mit einer Zentrifugalabtrennvorrichtung zum Separieren von einer im Wesentlichen unverzuckerte Bestandteile umfassenden Phase aus dem verzuckerten Rohsirup und mit einer Austragsvorrichtung zum Abscheiden der im Wesentlichen unverzuckerte Bestandteile umfassenden Phase aus der Zentrifugalabtrennvorrichtung separat von der im Wesentlichen Proteine und/oder Fette umfassenden Phase und separat von dem dann im Wesentlichen Glukose umfassenden restlichen Glukosesirup versehen ist. Alternativ oder zusätzlich kann die Anlage auch derart gestaltet sein, dass mit der Austragsvorrichtung sowohl die im Wesentlichen unverzuckerte Bestandteile umfassende Phase als auch der restliche Glukosesirup in einem einzelnen Volumenstrom aus der Zentrifugalabtrennvorrichtung abgeführt wird.

Durch eine Integration der genannten erfindungsgemäßen Zentrifugalabtrennvorgänge in einer einzigen Zentrifugalabtrennvorrichtung, insbesondere in Gestalt eines Dekanters, ist eine insgesamt besonders kostengünstige und prozesstechnisch besonders gut einzubindende Lösung geschaffen.

Die erfindungsgemäße Anlage ist ferner vorteilhaft dadurch weitergebildet, dass die Zentrifugalabtrennvorrichtung und insbesondere der Dekanter mit einer Schälscheibeneinrichtung zum Austragen des im Wesentlichen Glukose umfassenden restlichen Glukosesirups versehen ist. Eine derartige Einrichtung mit einer Schälscheibe ist insbesondere deshalb besonders gut zum Austragen des restlichen Glukosesirups geeignet, weil sie sehr variabel verstellbar ist. Dies ist insofern besonders wichtig, weil die Qualität und Zusammensetzung des industriell zu verarbeitenden Rohsirups sehr unterschiedlich sein kann. Trotz der verschiedenen Sirupqualitäten und -arten ist bei einer vorteilhaften Weiterbildung der Erfindung eine Schälscheibeneinrichtung vorgesehen, deren Schälscheibendurchmesser gezielt derart verstellbar ist, dass er zum Trommelinnendurchmesser in einem Verhältnis von zwischen zirka 0,64 bis zirka 0,76, besonders vorteilhaft von zwischen zirka 0,70 bis zirka 0,72 steht. Mit einem derartigen Schälscheibendurchmesser kann trotz verschiedener Durchsatzmengen und Sirupqualitäten eine hohe Qualität des abgeführten restlichen Glukosesirups erzielt werden.

Die erfindungsgemäße Abfuhr des restlichen Glukosesirups kann ferner vorteilhaft beeinflusst werden, indem die Zentrifugalabtrennvorrichtung und insbesondere der Dekanter mit einer Gegendruckeinrichtung zum Erzeugen eines Staudrucks am Austrag des im Wesentlichen Glukose umfassenden restlichen Glukosesirups versehen ist. Mit einer derartigen Erzeugung von Gegendruck am Austrag des restlichen Glukosesirups wird dessen Strömung innerhalb der Zentrifugalabtrennvorrichtung derart beeinflusst, dass es zu einem besonders vorteilhaften Austrag der im Wesentlichen Proteine und/oder Fette umfassenden Phase kommt. Bei dem erfindungsgemäßen Verfahren zur Herstellung von Glukose bildet die im Wesentlichen Proteine und/oder Fette umfassende Phase nämlich ein Flotat auf dem im Wesentlichen Glukose umfassenden restlichen Glukosesirup welches durch eine entsprechend beeinflusste Strömung des Glukosesirups in der Zentrifugalabtrennvorrichtung vorteilhaft zu jener Austragsvorrichtung gefördert werden kann, welche zum Abscheiden der im Wesentlichen Proteine und/oder Fette umfassenden Phase vorgesehen ist. Um während des Betriebs der erfindungsgemäßen Anlage einen möglichst stabilen Flotataustrag zu erzielen ist erfindungsgemäß ermittelt worden, dass der Gegendruck des restlichen Glukosesirups zwischen zirka 0,5 bar und zirka 3 bar betragen sollte.

Die erfindungsgemäße Zentrifugalabtrennvorrichtung ist ferner vorteilhaft an der Austragsvorrichtung zum Abscheiden der im Wesentlichen Proteine und/oder Fette umfassenden Phase mit einer Förderhilfe versehen. Mittels der Förderhilfe kann gezielt der Austrag von abgeschiedenem Fett und Eiweiß als unerwünschte Nebenprodukte für einen nachfolgenden Filtrationsprozess abgeschieden werden. Die Förderhilfe kann vorteilhaft als Schnecke bzw. Schneckenwendel gestalt sein, mit der ein so genannter "Wendelaustrag" möglich ist. Wie oben bereits erläutert ist bei der erfindungsgemäßen Zentrifugalabtrennvorrichtung die im Wesentlichen Proteine und/oder Fette umfassende Phase ein Flotat, welches auf dem im Wesentlichen Glukose umfassenden restlichen Glukosesirup aufschwimmt. Ein derartiges Flotat wird bei bekannten Zentrifugalabtrennvorrichtungen üblicherweise mittels eines Überlaufwehres abgeführt. Erfindungsgemäß ist hingegen zum Abführen der im Wesentlichen Proteine und/oder Fette umfassenden Phase ein Wendelaustrag vorgesehen, wie er bisher lediglich bei Zentrifugalabtrennvorrichtungen vorgesehen ist, welche zum Trennen eines Gemisches in eine Fest-, eine Flüssig-und eine zweite Fest-Phase vorgesehen ist. Mit anderen Worten wird bei der genannten erfindungsgemäßen Weiterbildung eine Fest-Flüssig-Fest-Zentrifugalmaschine zum Trennen eines Fest-Flüssig-Flüssig-Gemisches verwendet. Die erfindungsgemäße Zentrifugalabtrennvorrichtung ist dabei zum Abführen der im Wesentlichen unverzuckerten Bestandteile umfassenden Phase (welche gemäß der üblichen Definition eine Fest-Phase ist) vorteilhaft mit einer zweiten Schneckenwendel versehen, welche an der Austragsvorrichtung zum Ausscheiden der im Wesentlichen unverzuckerte Bestandteile umfassenden Phase angeordnet ist.

Um das Abscheiden der im Wesentlichen Proteine und/oder Fette umfassenden Phase weiter zu verbessern, ist an der zugehörigen Austragsvorrichtung vorteilhaft ferner ein Förderkonus vorgesehen, dessen Steigungswinkel in einen Bereich zwischen zirka 10° und zirka 20°, insbesondere zwischen zirka 15° und zirka 17° gestaltet ist. Ein derart geneigter Förderkonus führt selbst bei stark unterschiedlichen Qualitäten an Stärkelösung zu besonders guten Trennergebnissen. Erfindungsgemäß wurde ermittelt, dass bei in Bezug auf die Längsachse des Dekanters flacheren Steigungswinkel des Förderkonus sich die Abscheidezone des Flotats ungünstig verkürzt. Bei größeren Steigungswinkeln verschlechtert sich andererseits die Förderbarkeit auf den Förderkonus.

Alternativ oder zusätzlich kann die Qualität der erfindungsgemäßen Zentrifugaltrennung von Stärkelösung vor einem nachfolgenden Filtrationsprozess weiter verbessert werden, indem der genannte Förderkonus gezielt mit einer unebenen Oberfläche gestaltet ist. Eine solche Oberfläche kann beispielsweise aufgeraut sein oder mit Förderleisten versehen sein und dient in dieser Gestalt als Haftfläche für die am Förderkonus aufsteigende Phase bzw. das Flotat aus Fett und Eiweiß. Die Förderbarkeit dieses Flotats wird dadurch erhöht und insgesamt ein qualitativ hochwertigeres Trennergebnis erzielt.

Die Abtrennung von Flotat aus der erfindungsgemäßen Zentrifugalabtrennvorrichtung kann ferner vorteilhaft dadurch beeinflusst werden, dass die Zentrifugalabtrennvorrichtung und insbesondere der Dekanter an der Austragsvorrichtung zum Abscheiden der im Wesentlichen unverzuckerte Bestandteile umfassenden Phase mit einer Staueinrichtung zur Erhöhung des Förderdrucks der im Wesentlichen Proteine und/oder Fette umfassenden Phase aus der zugehörigen Austragsvorrichtung heraus versehen ist. Die Staueinrichtung ist vorteilhaft als Stauscheibe oder als Staublech gestaltet, welche/s von einem Schneckenkörper ausgehend, sich radial nach Außen im Wesentlichen quer bzw. längs der Schneckenachse erstreckt und eine Barriere bildet, welche verhindert, dass Flotat auf dem im Wesentlichen Glukose umfassenden restlichen Glukosesirup in die Richtung des Austrags der im Wesentlichen unverzuckerte Bestandteile umfassenden Phase schwimmen kann. Die Stauscheibe oder das Staublech ist besonders vorteilhaft derart groß im Durchmesser gestaltet, dass sie/es bis nahe an den Durchmesser einer Schneckenwendel des genannten Schneckenkörpers reicht. Das derart an der Stauscheibe bzw. dem Staublech angestaute Flotat erzeugt einen Förderdruck in Richtung der Austragsvorrichtung des Flotats.

Damit das angestaute Flotat bzw. die im Wesentlichen Proteine und/oder Fette umfassende Phase möglichst frei auf dem restlichen Glukosesirup zum zugehörigen Austrag abschwimmen kann, ist bei einer weiteren vorteilhaften Ausgestaltung der erfindungsgemäßen Anlage die Zentrifugalabtrennvorrichtung mit einer Schneckenwendel gestaltet, deren Schneckenkörper zwischen einem Einlass für den Stärkelösung und der Austragsvorrichtung zum Abscheiden der im Wesentlichen Proteine und/oder Fette umfassenden Phase im Wesentlichen keine Öffnung aufweist. Der genannte Schneckenkörper ist demnach im genannten Abschnitt mit einer glatten Oberfläche gestaltet, welche keine möglichen Staugebiete für Flotat am Schneckenkörper aufweist.

Die oben bereits erwähnte/n Schneckenwendel/n, welche als Förderhilfen zum Austragen der im Wesentlichen Proteine und/oder Fette umfassenden Phase sowie gegebenenfalls der im Wesentlichen unverzuckerte Bestandteile umfassenden Phase bei der erfindungsgemäßen Zentrifugalabtrennvorrichtung vorgesehen sein kann/können, wird/werden besonders vorteilhaft während des Betriebs in der zugehörigen Trommel in nacheilender Betriebsweise betrieben. "Nacheilend" bedeutet in diesem Zusammenhang, dass sich die Schneckenwendel langsamer dreht als die Trommel. Die nacheilende Betriebsweise unterstützt den Austrag von Flotat aus der erfindungsgemäßen Zentrifugalabtrennvorrichtung, denn der im Wesentlichen Glukose umfassende restliche Glukosesirup wandert in Richtung zu jener Austragsvorrichtung, welche zum Abscheiden der im Wesentlichen Proteine und/oder Fette umfassenden Phase dient.

Die erfindungsgemäße Zentrifugalabtrennvorrichtung ist ferner besonders vorteilhaft mit einer Trommel gestaltet, welche mit einem Durchmesser-Längen-Verhältnis von zwischen 1 zu 3 bis 1 zu 5, insbesondere von zirka 1 zu zirka 4 gestaltet ist. Eine derartige Dimensionierung der Trommel trägt zum Aufbau einer stabilen Flotatstrecke während des Betriebs der Vorrichtung bei. Das erzielte Klärergebnis ist entsprechend von hoher Qualität.

Die letztgenannten Details und damit erzielten Vorteile der erfindungsgemäßen Abtrennvorrichtung sind besonders von Bedeutung, wenn in der Prozesskette auf die Zentrifugaltrennung eine Filtrationsvorrichtung folgt, in der der im Wesentlichen Glukose umfassende restliche Glukosesirup weitergereinigt werden kann.

Die erfindungsgemäße Aufgabe ist schließlich auch mit einem Dekanter zum Trennen eines dreiphasigen Gemisches in einer Zentrifugaltrennung gelöst, welche eine erste Austragsvorrichtung zum Abscheiden einer sedimentierenden Phase aus dem Gemisch und eine zweite Austragsvorrichtung zum Abscheiden einer flotierenden Phase aus dem Gemisch aufweist. Die beiden Austragsvorrichtungen sind jeweils mit einer Schneckenwendel und einem zugehörigen Austragskonus gestaltet und jene Austragsvorrichtung, welche zum Abscheiden der sedimentierenden Phase dient, ist erfindungsgemäß mit einer Stauscheibe oder einem Staublech zum Aufstauen der flotierenden Phase vor der Austragsöffnung der sedimentierenden Phase versehen. Mit einem derartigen Dekanter und der/dem darin, wie oben erläutert, erfindungsgemäß angeordneten Stauscheibe bzw. Staublech wird erreicht, dass mit der eigentlich als Fest-Flüssig-Fest-Maschine arbeitenden Zentrifugaltrennvorrichtung auch ein Fest-Flüssig-Flüssig-Phasengemisch mit einer zumindest zähflüssigen flotierenden Phase verarbeitet werden kann.

Damit mit der genannten Maschine eine Trennung eines Fest-Flüssig-Flüssig-Gemisches in der erforderlichen Qualität erreicht werden kann, ist die/das erfindungsgemäß vorgesehene Stauscheibe bzw. Staublech besonders vorteilhaft mit einem Durchmesser von zirka 0,75 bis zirka 0,95 des Innendurchmessers einer zugehörigen Trommel des Dekanters gestaltet.

Im Übrigen ist die Aufgabe erfindungsgemäß durch Verwendung eines Dekanters gelöst, welcher mit einer Sedimentabscheidung und einer Flotatabscheidung versehen ist, um damit Glukose aus einer Stärkelösung zu gewinnen. Die Flotatabscheidung des Dekanters wird erfindungsgemäß zum Abscheiden von einer im Wesentlichen Proteine und/oder Fette umfassenden Phase aus der Stärkelösung verwendet und die Sedimentabscheidung des Dekanters zum Abscheiden einer im Wesentlichen unverzuckerte Bestandteile umfassenden Phase aus der Stärkelösung genutzt. Aus der Stärkelösung kann auf diese Weise ein im Wesentlichen Glukose umfassender restlicher Glukosesirup gewonnen werden, der in einem nachfolgenden Filtrationsprozess besonders gut verarbeitet werden kann und ferner eine besonders hohe Zuckerkonzentration aufweist.

Nachfolgend wird ein Ausführungsbeispiel eines erfindungsgemäßen Verfahrens zur Herstellung bzw. Gewinnung von Glukose aus einer Stärkelösung anhand der beigefügten schematischen Zeichnungen näher erläutert. Es zeigt:
Fig. 1 ein Ablaufdiagramm eines erfindungsgemäßen Verfahrens zur Herstellung von Glukose aus einer Stärkelösung,
Fig. 2 einen Längsschnitt eines ersten Ausführungsbeispiels einer Zentrifugalabtrennvorrichtung, welche bei dem Verfahren gemäß Fig. 1 verwendet wird, und
Fig. 3 einen Längsschnitt eines zweiten Ausführungsbeispiels einer Zentrifugalabtrennvorrichtung, welche bei dem Verfahren gemäß Fig. 1 verwendet wird.

### Detaillierte Beschreibung des Ausführungsbeispiels

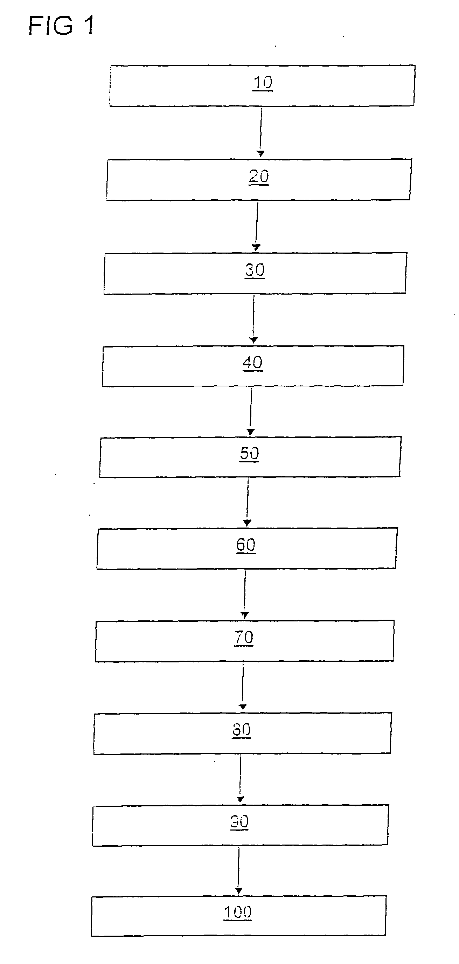

Das in Fig. 1 dargestellte Verfahren dient zum Herstellen von Glukose aus einer Stärkelösung, die beispielsweise aus Mais, Kartoffeln oder Weizen gewonnen worden ist. Die Stärkelösung wird zunächst in einem Prozessschritt 10 als eine Stärkesuspension aus Stärkemilch und Wasser hergestellt. Die Stärkesuspension wird nachfolgend in einem Prozessschritt 20 durch Zugabe von Dampf, Enzymen und/oder Säure verflüssigt. Durch ein weiteres Zugeben von Enzymen und Säure wird nachfolgend in einem Prozessschritt 30 eine Verzuckerung der Stärkelösung angeregt.

In einem Prozessschritt 40 wird die derart verzuckerte Stärkelösung als Rohsirup in einem Lagertank zwischengelagert und mit einem Rührwerk derart aufgerührt, dass aus dem Lagertank ein vergleichsweise homogenes Gemisch von Rohsirup entnommen werden kann, welches sowohl unlösliche Proteine und Fette, Glukose bzw. Dextrose als auch unverzuckerte Bestandteile aufweist.

Der entnommene Rohsirup wird in einem Prozessschritt 50 einer Zentrifugaltrennung unterworfen und dabei eine im Wesentlichen Proteine und/oder Fette umfassende Phase sowie getrennt von dieser eine im Wesentlichen unverzuckerte Bestandteile umfassende Phase abgeschieden. Mit der derartigen Zentrifugaltrennung wird ein Glukosesirup separiert, welcher im Wesentlichen Glukose umfasst. Dieser Glukosesirup wird in einem Prozessschritt 60 einer Filtration in einer Ultrafiltrationsvorrichtung unterworfen. Es folgt eine Reinigung in einer lonentauschervorrichtung gemäß dem dargestellten Prozessschritt 70 und schließlich eine Endreinigung gemäß einem Prozessschritt 80. Der derart gereinigte Glukosesirup wird in einem Prozessschritt 90 aufkonzentriert und schließlich wird ein fertiges Glukoseendprodukt durch ein Mischen verschiedener Glukose bzw. Dextrosequalitäten in einem Prozessschritt 100 hergestellt.

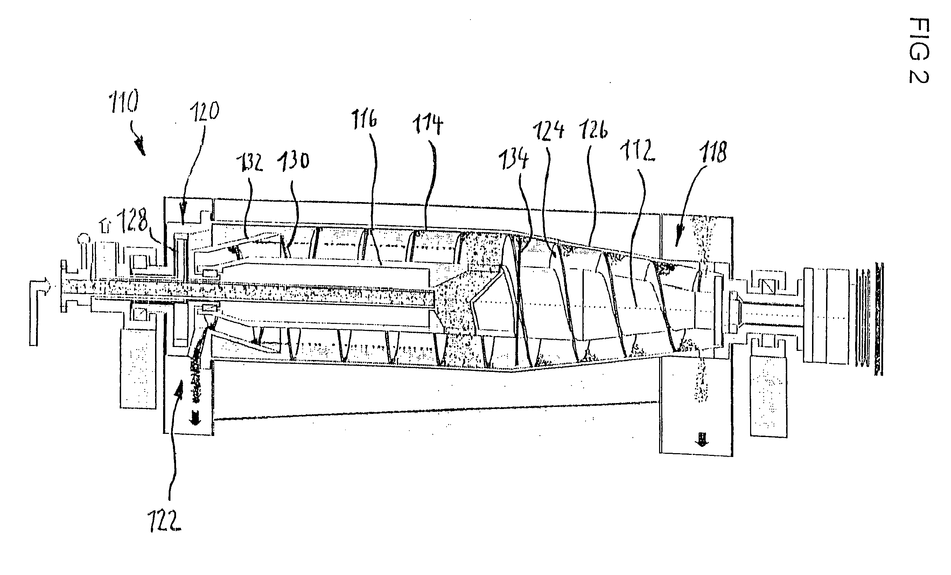

In Fig. 2 ist eine Zentrifugalabtrennvorrichtung 110 im Längsschnitt veranschaulicht, wie sie für den oben genannten Prozessschritt 50 der Zentrifugaltrennung verwendet wird. Die Zentrifugalabtrennvorrichtung 110 ist mit einer horizontalen Drehachse 112 gestaltet, um die eine Trommel 114 im Betrieb der Zentrifugalabtrennvorrichtung 110 mit hoher Drehzahl umläuft. In der Trommel 114 erstreckt sich längs der Drehachse 112 ein Schneckenkörper 116, der ebenfalls in Drehung versetzt werden kann.

An der Trommel 114 befinden sich insgesamt drei Vorrichtungen zum Austragen von jeweils einer Phase aus der in die Trommel 114 zugeführten verzuckerten Stärkelösung.

So ist eine Austragsvorrichtung 118 für eine im Wesentlichen unverzuckerte Bestandteile umfassende Phase sowie ein Austrag 120 für einen im Wesentlichen Glukose umfassenden restlichen Glukosesirup vorgesehen. Ferner ist eine Austragsvorrichtung 122 an der Trommel 114 angeordnet, welche zum Austragen von einer im Wesentlichen Proteine und/oder Fette umfassenden Phase vorgesehen ist.

Die Austragsvorrichtung 118 für die im Wesentlichen unverzuckerte Bestandteile umfassende Phase ist mit einer ersten Schneckenwendel 124 gestaltet, welche ein sich in der Trommel 114 an deren Innenseite anlagerndes Sediment entlang einem ersten Austragskonus 126 radial nach innen aus dem sich in der Trommel 114 befindlichen Rohsirup herausfördern kann. Der erste Austragskonus 126 bildet dabei einen bezogen auf Fig. 2 rechts angeordneten Endabschnitt der Trommel 114.

Der Austrag 120 für den im Wesentlichen Glukose umfassenden restlichen Glukosesirup ist mit einer Schälscheibeneinrichtung 128 gestaltet, die sich an der im ersten Austragskonus 126 gegenüberliegenden Endseite der Trommel 114 befindet. Die Schälscheibeneinrichtung 128 ist mit einem Schälscheibendurchmesser gestaltet, der zum Trommelinnendurchmesser in einem Verhältnis von zirka 0,71 steht.

Die Austragsvorrichtung 122 für die im Wesentlichen Proteine und/oder Fette umfassende Phase ist mit einer zweiten Schneckenwendel 130 gestaltet, welche sich an dem zur Schälscheibeneinrichtung 128 gerichteten Endbereich des Schneckenkörpers 116 befindet (bezogen auf Fig. 2 also am linken Endabschnitt des Schneckenkörpers 116). Der Durchmesser der zweiten Schneckenwendel 130 ist insgesamt kleiner gestaltet, als jener der ersten Schneckenwendel 124 und ist teilweise von einem zweiten Austragskonus 132 umgeben, der mit einem Endbereich in die sich in der Trommel 114 unter Zentrifugalkraft befindliche verzuckerte Stärkelösung eintaucht.

Durch die genannte Zentrifugalwirkung wird aus der Stärkelösung die im Wesentlichen Proteine und/oder Fette umfassende Phase aufgrund ihres vergleichsweise geringen spezifischen Gewichts radial nach innen bewegt und bildet dort ein Flotat, welches auf dem im Wesentlichen Glukose umfassenden restlichen Glukosesirup aufschwimmt. Dieses Flotat wird mit Hilfe der zweiten Schneckenwendel 130 entlang dem zweiten Austragskonus 132 aus der Zentrifugalabtrennvorrichtung 110 herausgefördert. Damit das Flotat dabei am zweiten Austragskonus 132 besser haftet, ist der zweite Austragskonus 132 mit einer aufgerauten Oberfläche gestaltet.

Der Austrag von Flotat bzw. der im Wesentlichen Proteine und/oder Fette umfassenden Phase ist ferner dadurch verbessert, dass innerhalb der ersten Schneckenwendel 124 eine Stauscheibe 134 ausgebildet ist. Die Stauscheibe 134 ist in jenem Bereich der ersten Schneckenwendel 124 angeordnet, wo diese aus dem im Wesentlichen zylindrischen Abschnitt der Trommel 114 in den Abschnitt des ersten Austragskonus 126 übergeht. Die Stauscheibe 134 ist dabei als eine sich quer zur Drehachse 112 erstreckende Kreisscheibe ausgebildet. Sie ist mit einem Durchmesser gestaltet, der zirka 0,9 des Innendurchmessers der zugehörigen Trommel 114 im Bereich der Anordnung der Stauscheibe 134 entspricht.

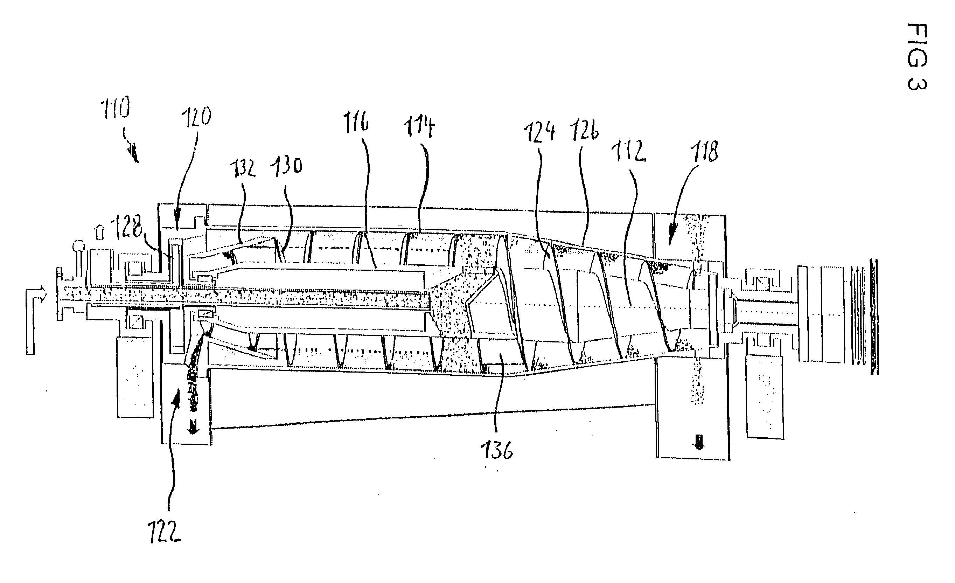

In Fig. 3 ist ein Ausführungsbeispiel einer erfindungsgemäßen Zentrifugalabtrennvorrichtung abgebildet, welche im Wesentlichen jener der Fig. 2 entspricht. Insbesondere sind bei der Zentrifugalabtrennvorrichtung der Fig. 3 der gleiche Austrag 120 für im Wesentlichen Glukose umfassenden restlichen Glukosesirup und die gleiche Austragsvorrichtung 122 für eine im Wesentlichen Proteine und/oder Fette umfassende Phase vorgesehen. Hinsichtlich der Austragsvorrichtung 118 für eine im Wesentlichen unverzuckerte Bestandteile umfassende Phase ist bei der Zentrifugalabtrennvorrichtung 110 gemäß Fig. 3 hingegen an Stelle einer Stauscheibe ein Staublech 136 vorgesehen.

Das Staublech 136 ist als Wehr gestaltet, welches sich zwischen zwei Gängen der ersten Schneckenwendel 124 befindet, und zwar in jenem Abschnitt der Schneckenwendel 124, an dem auch der im Wesentlichen zylindrische Abschnitt der Trommel 114 in den Abschnitt des ersten Austragskonus 126 übergeht. Das Staublech 136 erstreckt sich im Wesentlichen parallel zur Drehachse 112 radial nach außen und ragt dabei bis etwa 0,85 des Innendurchmessers der Trommel 114 in diese hinein.

### Bezugszeichenliste

- 10: Herstellen einer Stärkesuspension
- 20: Verflüssigen der Stärkesuspension
- 30: Verzuckerung der Stärkesuspension
- 40: Lagerung der verzuckerten Stärkesuspension
- 50: Abscheiden von einer im Wesentlichen Proteine und/oder Fette umfassenden Phase in einer Zentrifugalabtrennvorrichtung
- 60: Filtration in einer Ultrafiltrationsvorrichtung
- 70: Reinigen in einer Ionentauschervorrichtung
- 80: Endreinigung
- 90: Aufkonzentration
- 100: Herstellen des Glukoseendproduktes durch Mischen verschiedener Glukose- bzw. Dextrosequalitäten
- 110: Zentrifugalabtrennvorrichtung
- 112: Drehachse
- 114: Trommel
- 116: Schneckenkörper
- 118: Austragsvorrichtung für eine im Wesentlichen unverzuckerte Bestandteile umfassende Phase
- 120: Austrag für im Wesentlichen Glukose umfassenden restlichen Glukosesirup
- 122: Austragsvorrichtung für eine im Wesentlichen Proteine und/oder Fette umfassende Phase
- 124: erste Schneckenwendel
- 126: erster Austragskonus
- 128: Schälscheibeneinrichtung
- 130: zweite Schneckenwendel
- 132: zweiter Austragskonus
- 134: Stauscheibe
- 136: Staublech

## Patentansprüche

1. Verfahren zur Herstellung von Glukose aus einer Stärkelösung, mit den Schritten:
Verzuckerung der Stärkelösung (30) durch Zugabe von Enzymen und/oder Säure zu Rohsirup,
Separieren von einer im Wesentlichen Proteine und/oder Fette umfassenden Phase (50) aus dem Rohsirup in einer Zentrifugalabtrennvorrichtung (110) und
Abscheiden der im Wesentlichen Proteine und/oder Fette umfassenden Phase (50) aus der Zentrifugalabtrennvorrichtung (110) separat vom restlichen Rohsirup.

2. Verfahren nach Anspruch 1,
**gekennzeichnet durch** einen Schritt:
Separieren von einer im Wesentlichen unverzuckerte Bestandteile umfassenden Phase aus dem Rohsirup in einer Zentrifugalabtrennvorrichtung (110) und
Abscheiden der im Wesentlichen unverzuckerte Bestandteile umfassenden Phase aus der Zentrifugalabtrennvorrichtung (110) separat von der im Wesentlichen Proteine und/oder Fette umfassenden Phase und separat von dem dann im Wesentlichen Glukose umfassenden restlichen Glukosesirup.

3. Verfahren nach Anspruch 2,
**dadurch gekennzeichnet, dass** das Separieren der im Wesentlichen Proteine und/oder Fette umfassenden Phase (50) und der im Wesentlichen unverzuckerte Bestandteile umfassenden Phase in einer einzigen Zentrifugalabtrennvorrichtung (110), insbesondere in Gestalt eines einzigen Dekanters durchgeführt wird.

4. Verfahren nach einem der Ansprüche 1 bis 3,
**dadurch gekennzeichnet, dass** der restliche Glukosesirup, welcher im Wesentlichen Glukose umfasst, in einer der Zentrifugalabtrennvorrichtung (110) nachfolgenden Filtration (60) gereinigt wird.

5. Anlage zur Herstellung von Glukose aus einer Stärkelösung, mit
einer Zugabevorrichtung zum Zugeben von Enzymen und/oder Säure zur Stärkelösung, wodurch eine Verzuckerung der Stärkelösung zu Rohsirup angeregt wird, einer Zentrifugalabtrennvorrichtung (110) zum Separieren von einer im Wesentlichen Proteine und/oder Fette umfassenden Phase aus dem Rohsirup, und
einer Austragvorrichtung (122) zum Abscheiden der im Wesentlichen Proteine und/oder Fette umfassenden Phase aus der Zentrifugalabtrennvorrichtung (110) separat von dem restlichen Rohsirup.

6. Anlage nach Anspruch 5,
**gekennzeichnet durch** eine Zentrifugalabtrennvorrichtung (110) zum Separieren von einer im Wesentlichen unverzuckerte Bestandteile umfassenden Phase aus dem Rohsirup und
eine Austragvorrichtung (118) zum Abscheiden der im Wesentlichen unverzuckerte Bestandteile umfassenden Phase aus der Zentrifugalabtrennvorrichtung separat von der im Wesentlichen Proteine und/oder Fette umfassenden Phase und separat von dem dann im Wesentlichen Glukose umfassenden restlichen Glukosesirup.

7. Anlage nach Anspruch 6,
**gekennzeichnet durch** eine einzige Zentrifugalabtrennvorrichtung (110), insbesondere in Gestalt eines Dekanters, in der das Separieren der im Wesentlichen Proteine und/oder Fette umfassenden Phase (50) und der im Wesentlichen unverzuckerte Bestandteile umfassenden Phase gemeinsam durchführbar ist.

8. Anlage nach einem der Ansprüche 5 bis 7,
**dadurch gekennzeichnet, dass** die Zentrifugalabtrennvorrichtung (110) und insbesondere der Dekanter zum Austragen des im Wesentlichen Glukose umfassenden restlichen Glukosesirups mit einer Schälscheibeneinrichtung (128) versehen ist.

9. Anlage nach einem der Ansprüche 5 bis 8,
**dadurch gekennzeichnet, dass** die Zentrifugalabtrennvorrichtung (110) und insbesondere der Dekanter mit einer Gegendruckeinrichtung zum Erzeugen eines Staudrucks an einem Austrag (120) des im Wesentlichen Glukose umfassenden restlichen Glukosesirups versehen ist.

10. Anlage nach einem der Ansprüche 5 bis 9,
**dadurch gekennzeichnet, dass** die Zentrifugalabtrennvorrichtung (110) und insbesondere der Dekanter an der Austragsvorrichtung (122) zum Abscheiden der im Wesentlichen Proteine und/oder Fette umfassenden Phase mit einer Förderhilfe (130, 132) versehen ist.

11. Anlage nach einem der Ansprüche 5 bis 10,
**dadurch gekennzeichnet, dass** die Zentrifugalabtrennvorrichtung (110) und insbesondere der Dekanter an der Austragsvorrichtung (122) zum Abscheiden der im Wesentlichen Proteine und/oder Fette umfassenden Phase mit einem Förderkonus (132) mit einem Steigungswinkel von zwischen zirka 10° und zirka 20°, insbesondere von zirka 15° bis zirka 17° versehen ist.

12. Anlage nach einem der Ansprüche 5 bis 11,
**dadurch gekennzeichnet, dass** die Zentrifugalabtrennvorrichtung (110) und insbesondere der Dekanter an der Austragsvorrichtung (122) zum Abscheiden der im Wesentlichen Proteine und/oder Fette umfassenden Phase mit einem Förderkonus (132) versehen ist, dessen Oberfläche gezielt uneben gestaltet ist.

13. Anlage nach einem der Ansprüche 5 bis 12,
**dadurch gekennzeichnet, dass** die Zentrifugalabtrennvorrichtung (110) und insbesondere der Dekanter an der Austragsvorrichtung (118) zum Abscheiden der im Wesentlichen unverzuckerte Bestandteile umfassenden Phase mit einer Staueinrichtung (134, 136) zur Erhöhung des Förderdrucks der im Wesentlichen Proteine und/oder Fette umfassenden Phase aus der zugehörigen Austragsvorrichtung (122) heraus versehen ist.

14. Anlage nach einem der Ansprüche 5 bis 13,
**dadurch gekennzeichnet, dass** die Zentrifugalabtrennvorrichtung (110) und insbesondere der Dekanter mit einer Schneckenwendel (124, 130) gestaltet ist, deren zugehöriger Schneckenkörper (116) zwischen einem Einlass für die verzuckerte Stärkelösung und der Austragsvorrichtung (122) zum Abscheiden der im Wesentlichen Proteine und/oder Fette umfassenden Phase im Wesentlichen keine Öffnungen aufweist.

15. Anlage nach einem der Ansprüche 5 bis 14,
**dadurch gekennzeichnet, dass** die Zentrifugalabtrennvorrichtung (110) und insbesondere der Dekanter mit einer Schneckenwendel (124, 130) gestaltet ist, welche in einer Trommel (114) angeordnet ist und während des Betriebs in einer der Trommel (114) nacheilenden Betriebsweise rotierend betrieben wird.

16. Anlage nach einem der Ansprüche 5 bis 15,
**dadurch gekennzeichnet, dass** die Zentrifugalabtrennvorrichtung (110) und insbesondere der Dekanter mit einer Trommel (114) gestaltet ist, welche mit einem Durchmesser-Längen-Verhältnis von zirka 1 zu 4 bis zirka 1 zu 5 gestaltet ist.

17. Anlage nach einem der Ansprüche 5 bis 16,
**gekennzeichnet durch** eine Filtrationsvorrichtung, in der der im Wesentlichen Glukose umfassende restliche Glukosesirup weiter gereinigt werden kann.

18. Dekanter (110) zum Trennen eines dreiphasigen Gemisches in einer Zentrifugaltrennung mit einer ersten Austragsvorrichtung (118) zum Abscheiden einer sedimentierenden Phase aus dem Gemisch und einer zweiten Austragsvorrichtung (122) zum Abscheiden einer flotierenden Phase aus dem Gemisch, wobei die beiden Austragsvorrichtungen (118, 122) jeweils mit einer Schneckenwendel (124, 130) und einem zugehörigen Austragskonus (126, 132) gestaltet sind,
**dadurch gekennzeichnet, dass** die Austragsvorrichtung (118) zum Abscheiden der sedimentierenden Phase mit einer Staueinrichtung, insbesondere in Gestalt einer Stauscheibe (134) und/oder eines Staublechs (136) zum Aufstauen der flotierenden Phase vor der Austragsöffnung der sedimentierenden Phase versehen ist.

19. Dekanter nach Anspruch 18,
**dadurch gekennzeichnet, dass** die Stauscheibe (134) oder das Staublech (136) zirka 0,75 bis zirka 0,95 des Innendurchmessers einer zugehörigen Trommel (114) des Dekanters (110) in die Trommel (114) hineinragt.

20. Verwendung eines Dekanters (110), welcher mit einer Sedimentabscheidung (118) und einer Flotatabscheidung (122) versehen ist, zur Herstellung von Glukose aus einer Stärkelösung, wobei die Flotatabscheidung (122) des Dekanters (110) zum Abscheiden von einer im Wesentlichen Proteine und/oder Fette umfassenden Phase aus der Stärkelösung verwendet wird und die Sedimentabscheidung (118) des Dekanters (110) zum Abscheiden einer im Wesentlichen unverzuckerte Bestandteile umfassenden Phase aus der Stärkelösung verwendet wird.
